Europäisches Patentamt

European Patent Office

Office européen des brevets

(11) Publication number: **0 336 508**
**A1**

## EUROPEAN PATENT APPLICATION

(21) Application number: **89200825.1**

(22) Date of filing: **30.03.89**

(51) Int. Cl.4: **C12N 15/00 , C12N 9/72 , A61K 37/00**

(30) Priority: **06.04.88 US 178205**

(43) Date of publication of application:
**11.10.89 Bulletin 89/41**

(84) Designated Contracting States:
**AT BE CH DE ES FR GB GR IT LI LU NL SE**

(71) Applicant: **K.U. LEUVEN RESEARCH & DEVELOPMENT**
**Benedenstraat 59A Groot Begijnhof**
**B-3000 Leuven(BE)**

Applicant: **Collen, Désiré José**
**Schoonzichtlaan 20**
**B-3009 Winksele-Herent(BE)**

(72) Inventor: **Collen, Désiré José**
**Schoonzichtlaan 20**
**B-3009 Winksele-Herent(BE)**
Inventor: **Nelles, Lucien Georges Raymond**
**Geerdegemdries 21**
**B-2800 Mechelen(BE)**
Inventor: **Lijnen, Henri Roger**
**Acacialaan 50A**
**B-3020 Herent(BE)**
Inventor: **De Cock, Frans**
**Spuibeekweg 18**
**B-2850 Eerbergen(BE)**
Inventor: **Van Hoef, Berthe**
**Bergstraat 97**
**B-3200 Kessel-Loo(BE)**
Inventor: **Stassen, Jean-Marie**
**Josef Ravoetstraat 5**
**B-3010 Wilsele(BE)**

(74) Representative: **Prins, Hendrik Willem et al**
**Octrooibureau Arnold & Siedsma**
**Sweelinckplein, 1**
**NL-2517 GK The Hague(NL)**

(54) Recombinant human single-chain urokinase-type plasminogen activator mutant produced by site-specific mutagenesis of lysine 158 to histidine 158.

(57) The invention relates to a recombinant human single chain urokinase-type plasminogen activator mutant produced by site specific mutagenesis of $Lys^{158}$ to $His^{158}$, and to its therapeutical use.

FIG.3A I

FIG.3B I

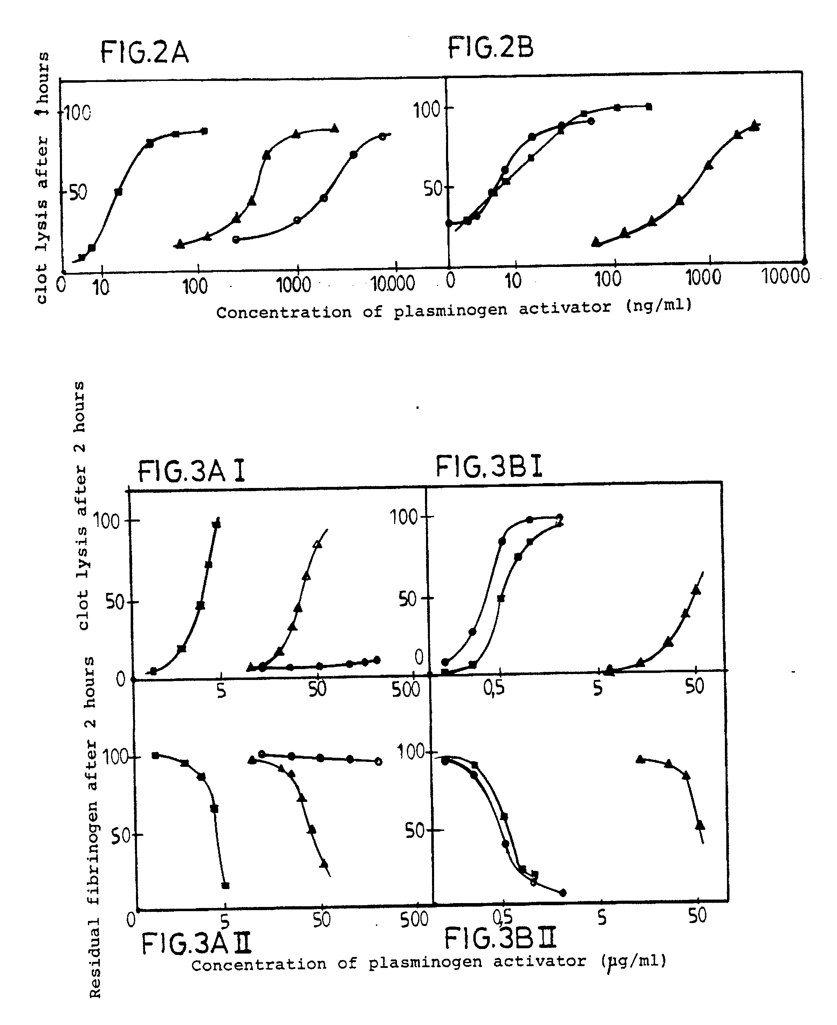

FIG.3A II

FIG.3B II

clot lysis after 2 hours

Residual fibrinogen after 2 hours

Concentration of plasminogen activator (μg/ml)

## Recombinant human single-chain urokinase-type plasminogen activator mutant produced by site-specific mutagenesis of lysine 158 to histidine 158

Urokinase-type plasminogen activator (u-PA) has been purified as a single-chain molecule (scu-PA) from several natural sources, including urine, plasma or conditioned cell culture media (for references, see Lijnen, H.R. et al (1987) Sem. Thromb. Hemost. 13, 152-159). In addition, the cDNA encoding u-PA has been cloned and expressed in E. coli (Holmes, W.E. et al (1985) Biotechnology 3, 923-929) and in Chinese hamster ovary cells (Nelles, L. et al (1987) J. Biol. Chem. 262, 5682-5689). A two-chain derivative of scu-PA (tcu-PA, urokinase) is generated by specific cleavage of the $Lys^{158}$-$Ile^{159}$ peptide bond in scu-PA by plasmin, scu-PA, in contrast to tcu-PA, has a very low amidolytic activity towards low $\underline{M}_r$ synthetic substrates (Wun, T.C. et al (1982) J. Biol. Chem. 257. 7262-7268).

scu-PA has a significant degree of fibrin-specificity in vivo, its use for coronary thrombolysis in man is still associated with extensive systemic plasminogen activation and fibrinogen breakdown in almost 50 percent of the patients (Van de Werf, F., Nobuhara, M., Collen, D. (1986) Ann. Int. Med. 104, 345-348). The requirement of a high dose for therapeutic efficacy relative to that of urokinase could be due either to a lower intrinsic plasminogen activating potential of scu-PA or to the necessity, for enzymatic activity, of in vivo conversion of the single chain to the two chain form. The systemic fibrinogen degradation, an undesirable side effect which may predispose to bleeding may be an intrinsic property of the single chain form or alternatively be caused by systemic plasminogen activation via generated tcu-PA. Based on the hypothesis that scu-PA is a highly fibrin-selective thrombolytic agent and that systemic fibrinogen breakdown is due to plasmin-mediated generation of tcu-PA, we have initiated investigations for rscu-PA mutants for use as more fibrin-specific thromolytic agents which does not posses the afore-mentioned adverse side effect.

When $Lys^{158}$ was converted to Glu or Gly by site directed mutagenesis of rscu-PA, mutants were obtained which are resistant to cleavage by plasmin (Nelles, 1987). These mutants still activated plasminogen, albeit with a catalytic efficiency ($k_2/k_m$) which was 20-fold lower than that of wild type scu-PA. Although these findings confirmed that these scu-PA mutants had intrinsic plasminogen activating potential, rscu-PA-$Glu^{158}$ was found to have a catalytic efficiency which has less than 1 percent of that of its two chain derivative obtained by hydrolysis with Endoproteinase Glu-C (Lijnen, H.R. et al (1988) Eur. J. Biochem. 172, 185-188). Furthermore, rscu-PA-$Glu^{158}$ had a very low specific activity on fibrin plates (1 percent of that of rscu-PA) and did not produce significant thrombolysis in a plasma clot lysis system in vitro at a concentration which was 20-fold higher than the concentration of wild type rscu-PA which produced 50 percent lysis. These findings strongly suggest that amplification of the fibrinolytic process by conversion of scu-PA to tcu-PA is responsible for the discrepant behavior of rscu-PA and rscu-PA-$Glu^{158}$ and consequently that tcu-PA generation is an essential step of the mechanism of clot dissolution with scu-PA.

The behavior of rscu-PA in a plasma milieu in vitro may however not be indicative of its physiological mechanism of action. Indeed, dose-response curves of rscu-PA in plasma clot lysis systems in vitro show a marked threshold phenomenon (Collen, D. et al(1986b) Thromb. Haemost. 56, 35-39), whereas the dose response curves for thrombolysis in vivo, both in rabbit and dog models, are linear (Collen, D. (1986c) Circulation 74, 1066-1070). Furthermore, rscu-PA-$Glu^{158}$ and rscu-PA-$Gly^{158}$ appear to have some thrombolytic activity in vivo, but only at high doses, which precludes a quantitative comparison with that of wild-type rscu-PA. The invention has for its object to provide a rscu-PA mutant suitable for use as a highly fibrin-specific thromolytic agent which does not posses the undesired side effect of scu-PA. Another object of the invention is the provision of a recombinant DNA molecule comprising a coding sequence for the rscu-PA mutant. A further object of the invention is a mammalian expression vector containing the recombinant DNA molecule loding for said rscu-PA mutant. Still another object of the invention is to provide a transfected mammalian cell line which comprises the recombinant DNA molecule.

The invention relates further to a method for the preparation of the rsu-PA mutant, to a medical composition with thrombolytic activity comprising this rsu-PA mutant, and to a method for the treatment of patients suffering from thrombo-embolic disease.

The recombinant single chain urokinase-type plasminogen activator mutant obtained by site-specific mutagenesis of $Lys^{158}$ for $His^{158}$ rscu-PA-$His^{158}$) has these desired properties.

For medical use rscu-PA-$His^{158}$ is included in for instance a conventional intravenous infusion liquid.

rscu-PA-$His^{158}$ was found to activate plasminogen directly following Michaelis-Menten kinetics with a catalytic efficiency $k_2/k_m$ of 0,62 $mM^{-1}s^{-1}$ which is similar to that of wild type rscu-PA (0,64 $mM^{-1}s^{-1}$). However, rscu-PA-$His^{158}$ had a much lower affinity for plasminogen than rscu-PA ($k_m$ of 80 $\mu M$ and 1,1 $\mu M$

respectively) but a much higher turnover rate constant ($k_2$ of 0,05 $s^{-1}$ as compared to 0.0007 $s^{-1}$). Plasmin did not hydrolyze the $His^{158}$-$Ile^{159}$ peptide bond in rscu-PA-$His^{158}$ and thus did not produce a urokinase derivative with a higher catalytic efficiency as compared to the single chain precursor. Plasmin, like thrombin, did hydrolyze the $Arg^{156}$-$Phe^{157}$ peptide bond and also yielded a two chain derivative with kinetic constants very similar to those of scu-PA-$His^{158}$.

Although rscu-PA-$His^{158}$ had a catalytic efficiency comparable to that of rscu-PA, its specific activity on fibrin plates was 30 times lower and its fibrinolytic potency in a plasma clot lysis system in vitro 17 times lower than that of rscu-PA. However, in an in vivo rabbit preparation for quantitative thrombolysis rscu-PA and rscu-PA-$His^{158}$ are equipotent, requiring about 1 mg/kg over 4 hours for 30 percent clot lysis. Both compounds displayed similar linear dose-response curves of thrombolysis which are markedly at variance with the strongly non-linear dose-response curves observed in the in vitro plasma clot lysis system. The discrepancy between strongly non-linear in vitro versus linear in vivo dose response curves does not appear to be a species specific phenomenon. Indeed non-linear dose-response curves in a plasma milieu in vitro have been observed with scu-PA in the plasma of many species (Lijnen, H.R. (1984) Thromb. Haemost., 52, 31-33), whereas a linear in vivo dose response has been found both in rabbits with jugular vein thrombosis (Collen 1986c) and in dogs with femoral vein thrombosis.

The finding that rscu-PA and rscu-PA-$His^{158}$ are equipotent thrombolytic agents suggests that fibrin-specific thrombolysis in vivo is caused by the single chain form of u-PA and that amplification of the fibrinolytic process by conversion of scu-PA to tcu-PA is not a major contributing factor to thrombus dissolution. Our present study indicates that the results of in vitro experiments with scu-PA, even in a plasma milieu, are not relevant for its physiological mechanism of action. Indeed, the dose-response curves and thrombolytic potency of scu-PA and its derivatives in vitro are not comparable with in vivo results. In addition scu-PA and t-PA display no useful synergism in vitro (Collen 1986b) whereas they are significantly synergistic in vivo (Collen 1986c, 34). Finally, the comparable in vivo thrombolytic potency of rscu-PA-$His^{158}$ and rscu-PA could not have been anticipated from their markedly different in vitro behaviour.


scu-PA-$His^{158}$, its construction and expression.

Wild type recombinant scu-PA and rscu-PA-$Glu^{158}$ were obtained by expression of cDNA in CHO cells (Nelles et al). Natural scu-PA (nscu-PA) was obtained from Sandoz AG (Basel, Switzerland) and recombinant scu-PA for in vivo experiments in rabbits from Grünenthal (Aachen, W. Germany). Two chain derivatives of rscu-PA and rscu-PA-$Glu^{158}$ were obtained by digestion with plasmin or with Endoproteinase Glu-C as described (Lijnen et al 1988). Human plasminogen, plasmin and fibrinogen were prepared and characterized as described (Lijnen, H.R. (1986a) J. Biol. Chem. 261, 1253-1258).

Aprotinin (Trasylol[R]) was from Bayer (Leverkusen, F.R.G.), and Pyroglu-Gly-Arg-p-nitroanilide (S-2444) and D-Val-Leu-Lys-p-nitroanilide (S-2251) from KabiVitrum (Brussels, Belgium). The International Reference Preparation of Urokinase (66/46) was obtained from the National Institute for Biological Standards and Control (London, U.K.). Restriction enzymes and DNA modifying enzymes were from New England Biolabs (Boston, MA), Boehringer Mannheim (Mannheim, F.R.G.) or Gibco/BRL (Gent, Belgium).

Mutagenesis of the triplet encoding $Lys^{158}$ to encode His was performed using a Sau3AI restriction fragment (nucleotides 399-732) of the scu-PA-cDNA (Holmes, 1985), which was inserted in M13mp18 DNA (Yannish-Perron, C., et al (1985) Gene 33, 103-118), in such a way that the single stranded phage DNA contained the coding strand. Oligonucleotide directed mutagenesis, was performed as described (Nelles et al 1987) using the oligodeoxy-nucleotide 5′-CCCCAATAATGTGAAACGGGGCC13′. Mutant plaques were identified by differential plaque hybridization with the ($^{32}$P)-labeled oligonucleotide and the sequence was confirmed by the dideoxynucleotide chaintermination method (Sanger, F. et al (1977) Proc. Natl. Acad. Sci. USA 74, 5463-5467) of the DNA from positive plaques. To reconstruct the complete rscu-PA cDNA, a PstI-EcoRI restriction fragment (nucleotides 439-621), containing the mutation site, was isolated from the mutant M13 phage DNA and substituted for the wild-type sequence in pULscu-PA (Nelles, 1987). The complete coding sequence of the mutant was then transferred to the eukaryotic expression vector pSV328DHFR.

The expression plasmid was then used to transfect DHFR deficient CHO cells (Urlaub, G. et al (1980) Proc. Natl. Acad. Sci. USA 77, 4216-4220) with the calcium phosphate coprecipitation method (Graham, L.F. et al (1973) Virology 52, 456-467). Colonies of DHFR$^+$ CHO cells, secreting u-PA related antigen, were selected for large scale preparation of protein as described (Nelles, 1987). Celles were grown at 37° C in 850 $cm^2$ roller bottles (Becton & Dickinson, Oxnard, CA) in 250 ml MEMα medium supplemented with 10% fetal calf serum and 25 mM Hepes buffer, pH 7.3. At confluency, the MEMα medium was replaced by 350 ml optimem (Gibco/BRL, Gent, Belgium), supplemented with 5 mM Hepes buffer pH 7,3, 10 μg/ml insulin

3

(Gibco/BRL), 5 μg/ml transferrin (Gibco/BRL), 1X non essential amino acids (Gibco/BRL), 1X sodium pyruvate (Gibco/BRL), 100 U/ml Penicillin/Streptomycin (Gibco/BRL), 2 mM L-glutamine (Gibco/BRL) and 20 KIU/ml aprotinin (Bayer, Leverkusen, F.R.G.). Conditioned medium was harvested 3 times after 2 day incubation periods.

Purification of rscu-PA-His[158]

The rscu-PA mutant was purified from conditioned cell culture media by chromatography on Zin chelate-Sepharose followed by immunoadsorption on an insolubilized murine monoclonal antibody against u-PA (MA-4DIE8) (Nelles, 1987). The fractions containing u-PA related antigen were pooled and dialyzed against 0.3 M NaCl-0.02 M Tris.HCl buffer pH 7.4, containing 0.01% Tween 80 and 10 KIU/ml aprotinin. The starting concentrations of u-PA related antigen in pooled conditioned media from CHO cells transfected with cDNA encoding rscu-PA-His[158] were 0.86 ± 0.4 mg/l (mean ± SD, n = 4). Chromatography of 20 to 30 liter batches on Zin chelate-Sepharose yielded a 100-fold volume reduction with a nearly complete recovery. After immunoadsorption, the overall yield was 0.75 ± 0.15 mg/l. With 10 KIU/ml of aprotinin added to the cell culture media and the buffers, the mutant was obtained primarily in the single chain form. Before use, the proteins were chromatographed on benzamidine-Sepharose to eliminate traces of two cahin derivatives and aprotinin was removed by extensive washing on Centricon 30 microconcentrators (Amicon, Danvers MA). After chromatography on benazmidine-Sepharose, the baseline specific activity (S-2444) was 800 IU/mg.

Characterization of rscu-PA-His[158]

NH₂-terminal amino acid sequence analysis on thrombin-treated or plasmin-treated samples was performed at the Department of Biochemistry, University of Vermont, Burlington, VT (Courtesy of Dr. K.G. Mann), using described methodology (Hunkapiller, M.W., Hewick, R.M., Dreyer, W.J., Hood, L.E. (1983) Methods Enzymol. 91, 399-413). rscu-PA-His[158] (final concentration 5 μM) in TNT buffer at 37°C were treated with two successive additions of hum α-thrombin (1 percent on a molar basis each) for 15 min each, followed by neutralization of thrombin by addition of D-Ile-Pro-Arg-CH₂Cl (final concentration $10^{-4}$ M).

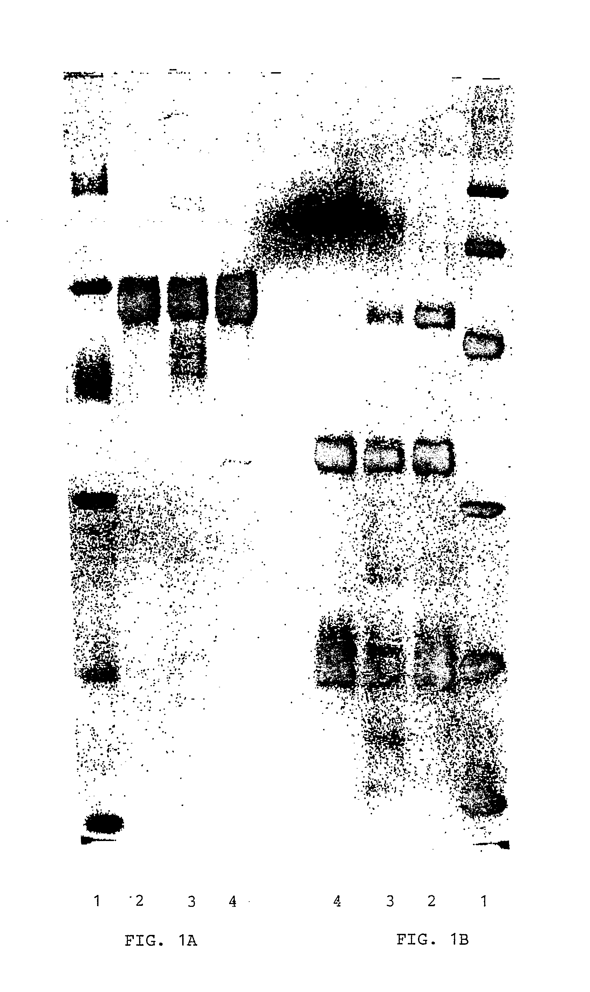

The amino acid composition (not shown) was in good agreement with that derived from the known sequence. NH₂-terminal amino acid sequence analysis of thrombin-treated samples revealed a double sequence. One was the NH₂- terminal sequence (Ser-Asn-Glu-Leu-His-Gln-Val-), which confirms that the translation product of the transfected cDNA was correctly processed by the CHO cells. The second sequence, detected in yields equal or slightly lower than the first sequence, corresponded with the expected sequence of the COOH-terminal chain generated by thrombin cleavage of the Arg[156]-Phe[157] peptide bond, namely Phe-His-Ile-Ile-Gly-Gly-. SDS-PAGE under non-reducing conditions was performed on 12% gels according to Laemmli (Laemmli, U.K. (1970) Nature 227, 680-685). Conversion of single chain to two chain molecules was monitored on 10-15% gradient gels after reduction with DTE using the Phast-System™ (Pharmacia, Uppsala, Sweden). rscu-PA-His[158] migrated as a single main band on SDS-PAGE, with $M_r$ about 54,000 (Fig. 1). u-Pa concentrations were determined by a specific ELISA (Darras, V. et al (1986) Thromb. Haemost. 56, 411-414) or by amino acid analysis on a Beckman 119 CL amino acid analyzer after 20 h hydrolysis in 6 M HCl at 110°C, in vacuo. Specific activities were determined on fibrin plates (Astrup, T. et al (1952) Arch. Biochem. Biophys. 40, 346-351) by comparison with the International Reference Preparation for urokinase. The specific activities on fibrin plates were 60,000 IU/mg for rscu-PA, 112,000 IU/mg for rtcu-PA, 2,100 IU/mg for rscu-PA-His[158], and 3,000 IU/mg for rtcu-PA-His[158].

Treatment of rscu-PH-His[158] with Plasmin or Thrombin

rscu-PH-His[158] (final concentration about 2 μM) in TNT buffer at 37°C was treated with plasmin (final concentration 2.5, 5 or 10 percent on a molar basis). At timed intervals (0-20 min), samples were diluted 80-fold in TNT buffer containing 0.3 mM S-2444 to measure urokinase activity, which was expressed in IU by comparison with the International Reference Preparation for urokinase. Plasmin caused a time- and concentration-dependent conversion of rscu-PA to amidolytically active urokinase, with a maximal specific activity of 80,000 IU/mg. No significant generation of active urokinase was observed for rscu-PA-His[158].

Reduced SDS-PAGE following treatment with 7 percent plasmin (molar ratio) respectively for 30 min (Fig. 1) shows nearly complete conversion from a one chain to a two chain molecule both for rscu-PA and rscu-PA-His[158], but the conversion of rscu-PA-His[158] was not associated with generation of amidolytic activity. For further analysis, rtcu-PA-His[158] was obtained by treatment of rscu-PA-His[158] (10 $\mu$M)with plasmin (7 percent on a molar basis) for 35 min at 37° C. Because rtcu-PA-His[158] did not bind significantly to benzamidine Sepharose (over 95 percent of protein in the breakthrough), it was used as such after inhibition of plasmin with aprotinin (2000 KIU/ml) followed by removal of excess aprotinin by extensive washing in a Centricon 30 microconcentrator.

NH$_2$-terminal amino acid sequence analysis of plasmin-treated rscu-PA-His[158] revealed, in addition to the NH$_2$ terminal amino acid sequence Ser-Asn-Glu-Leu-His-Gln-Val-, the presence of one major sequence Phe-His-Ile-Ile-Gly-Gly, which indicates that rscu-PA-His[158] is cleaved by plasmin at the Arg[156]-Phe[157] peptide bond (Tabel 1B).

rscu-PA-His[158] (final concentration about 20 $\mu$M) was also treated with thrombin (1 percent on a molar basis) in TNT buffer at 37° C for 20 min and urokinase activity was measured with S-2444 as described above. The treatment with thrombin also resulted in cleavage at Arg[156] Table 1A) and was associated with nearly complete conversion from one chain to two chain molecules as revealed by reduced SDS-PAGE (Fig. 1). No generation of amidolytically active urokinase with thrombin was observed.

EP 0 336 508 A1

## Table I

NH$_2$-terminal amino acid sequence of the COOH-terminal chain of rscu-PA-His[158] after treated with thrombin or plasmin.

| | | Cycle | | | | | |
|---|---|---|---|---|---|---|---|
| | | 1 | 2 | 3 | 4 | 5 | 6 |
| **A. Thrombin treated** | | | | | | | |
| Wild type | Expected[a] | Phe[157] | Lys[158] | Ile[159] | Ile[160] | Gly[161] | Gly[162] |
| rscu-PA-His[158] | | | | | | | |
| | Amino acid | Phe | His | Ile | Ile | Gly | Gly |
| | Yield | 73 | 39 | 58 | 56 | 64 | 56 |
| **B. Plasmin treated** | | | | | | | |
| Wild type | Expected[a] | Ile[159] | Ile[160] | Gly[161] | Gly[162] | Glu[163] | Phe[164] |
| rtcu-PA-His[158] | | | | | | | |
| | Amino acid | Phe | His | Ile | Ile | Gly | Gly |
| | Yield | 151 | 33 | 83 | 109 | 88 | 135 |

Yields obtained at each cycle are expressed in pmol and were obtained from analysis of 90 pmol thrombin-treated or 200 pmol plasmin-treated rscu-PA-His[158].
[a]The expected sequence for the COOH-terminal chain of wild-type rscu-PA treated with thrombin or plasmin is taken from ref 3.

Kinetics of the activation of Glu-plasminogen and Lysplasminogen by rscu-PA-His[158] and rtcu-PA-His[158]

Activation of Glu-plasminogen (10-88 µM) by rscu-PA-His[158] (33 nM) or by rtcu-PA-His[158] (33 nM) was measured at 37°C in TNT buffer. At timed intervals (0-5 min) samples were removed and generated plasmin measured with the chromogenic substrate S-2251 (final concentration 1 mM) after 20-fold dilution of the sample. Activation of Lys-plasminogen (1-10 uM) by rscu-PA-His[158] 17 nM) or by rtcu-PA-His[158] (17 nM) was measured by incubation of plasminogen in TNT buffer at 37°C. The generated plasmin at different time intervals was measured with S-2251 as described above. Initial activation rates were obtained from plots of the concentration of generated plasmin versus activation time. Kinetics of plasminogen activation by rscu-PA and rtcu-PA were determined as previously described (Collen 1986[b], Lijnen 1986[b]).

Activation of Glu-plasminogen or Lys-plasminogen by rscu-PA, rscu-PA-His[158], rtcu-PA and rtcu-PA-His[158] obeys Michaelis-Menten kinetics, as evidenced by linear double reciprocal plots of the initial activation rate versus the plasminogen concentration (not shown). The kinetic constants, obtained by linear regression analysis, are summarized in Tabel II. The kinetic analyses were carried out with two independent preparations of each u-PA moeity, with very similar results.

| | $K_m$ ($\mu M$) | $k_2$ ($s^{-1}$) | $k_2/K_m$ ($\mu M^{-1} s^{-1}$) | $r$ |
|---|---|---|---|---|
| **A. Glu-plasminogen** | | | | |
| rscu-PA | 1.10 | 0.0007 | 0.00064 | 0.996 |
| rtcu-PA | 80 | 3.7 | 0.046 | 0.998 |
| rscu-PA-His[158] | 80 | 0.05 | 0.00062 | 0.998 |
| rtcu-PA-His[158] | 90 | 0.05 | 0.00055 | 0.999 |
| **B. Lys-plasminogen** | | | | |
| rscu-PA | 0.7 | 0.0043 | 0.0061 | >0.99 |
| rtcu-PA | 2.8 | 5.8 | 2.1 | >0.99 |
| rscu-PA-His[158] | 13 | 0.05 | 0.0038 | 0.999 |
| rtcu-PA-His[158] | 6.7 | 0.038 | 0.0057 | 0.999 |

r: correlation coefficient obtained by linear regression analysis with at least 6 experimental points.

$^{125}$I-labeled fibrin clot lysis in a solution of Glu-plasminogen

$^{125}$I-labeled clots of human fibrin were prepared by addition of CaCl₂ (concentration 50 mM) and thrombin (final concentration 2 NIH units/ml) to purified fibrinogen (final concentration 3 mg/ml) containing approximately 250.000 cpm/ml of $^{125}$I-labeled fibrinogen) in TNT buffer. After incubation at 37°C for 60 min in silicon tubings (internal diameter 4 mm), pieces of about 1.5 cm length were cut and the fibrin clots were extensively washed in 0.15 M NaCl. Purified $^{125}$I-labeled fibrin clots were then incubated at 37°C in TNT buffer containing 1 KIU/ml aprotinin and 1.5 uM Glu-plasminogen, and lysis of the clots following addition of rscu-PA, rscu-PA-Glu$^{158}$, rscu-PA-His$^{158}$ and their two chain derivatives (concentration range 0.5-100 μg/ml) was measured for 1 hour at 15 min intervals from the release of radioactivity from the clot into the buffer. The concentration of u-PA yielding 50 percent clot lysis in 1 hour was determined from plots of the degree of lysis (in percent) obtained after 1 hour versus the concentration of plasminogen activator used.

For all u-PA moieties evaluated, time- and concentration-dependent lysis of $^{125}$I-labeled fibrin clots immersed in Glu-plasminogen was obtained. Concentrations of u-PA, required to obtain 50 percent fibrin clot lysis in 1 hour in Glu-plasminogen solutions were 0.016 μg/ml for rscu-PA, 2 μg/ml for rscu-PA-Glu$^{158}$, 0.3 μg/ml for rscu-PA-His$^{158}$, 0.007 μg/ml for rtcu-PA, 0.007 μg/ml for rtcu-PA-Glu$^{158}$ and 0.75 μg/ml for rtcu-PA-His$^{158}$ (Fig. 2).

Lysis of a $^{125}$-fibrin labeled hum plasma clot immersed in citrated human plasma

Lysis of a $^{125}$I-fibrin labeled plasma clot by rscu-PA (final concentration 0-5 μg/ml), rtcu-PA (0-2 μg/ml), rscu-PA-Glu$^{158}$ (0-100 μg/ml), rtcu-PA-Glu$^{158}$ (0-100 μg/ml), rscu-PA-His$^{158}$ (0-50 μg/ml) or rtcu-PA-His$^{158}$ - (0-50 μg/ml) was measured over 4 hours as previously described (Lijnen, 1986$^a$). The concentration of u-PA, required to obtain 50 percent clot lysis in 2 hours was determined from plots of the percent lysis versus the concentration of u-PA. rscu-PA, rscu-PA-Glu$^{158}$ and rscu-PA-His$^{158}$ and their two chain derivatives rtcu-PA, rtcu-PA-Glu$^{158}$ and rtcu-PA-His$^{158}$ induced a time- and concentration-dependent lysis of a $^{125}$I-fibrin labeled plasma clot immersed in human plasma (not shown). Fifty percent clot lysis in 2 hours (Fig. 3 AI en BI panels) was obtained with concentrations of 2.1 μg/ml for rscu-PA, > 200ug/ml for rscu-PA-Glu$^{158}$ and 35 μg/ml for rscu-PA-His$^{158}$. At these concentrations corresponding residual fibrinogen levels after 2 hours were 80, 100 and 60 percent for rscu-PA, rscu-PA-Glu$^{158}$ and rscu-PA-His$^{158}$ respectively (Fig. 3 AII and BII).

Fifty percent lysis in 2 hours was obtained with concentrations of 0.5 μg/ml for rtcu-PA, 0.5 μg/ml for rtcu-PA-Glu$^{158}$ and 50 μg/ml for rtcu-PA-His$^{158}$ (Fig. 3, upper panels) with corresponding residual fibrinogen levels of 55 , 40 and 50 percent respectively (Fig. 3 AII and BII).

Thrombolysis in a rabbit jugular vein thrombosis model

The in vivo thrombolytic properties of rscu-PA-His$^{158}$ were compared to those of natural scu-PA (Sandoz AG, Basel, Switzerland) and recombinant scu-PA (Grünenthal AG, Aachen, FRG) in the raabit jugular vein thrombosis model as previously described (Collen, D. et al (1983) J. Clin. Invest. 71, 368-376). $^{125}$I-fibrin labeled jugular vein thrombi were aged for 30 minutes. The plasminogen activator was administered via a contralateral marginal ear vein as a 10 percent bolus injection, followed by continuous infusion of the remaining 90 percent dose over four hours. Thrombolysis was quantitated 30 minutes after the end of the infusion by determination of the residual radioactivity in the jugular vein segment. Blood samples were drawn into 0.01 M citrate for determinations of residual fibrinogen (clotting rate assay), $\alpha_2$-antiplasmin (chromogenic substrate assay) and u-PA related antigen levels as previously described (Darras et al, Collen 1983).

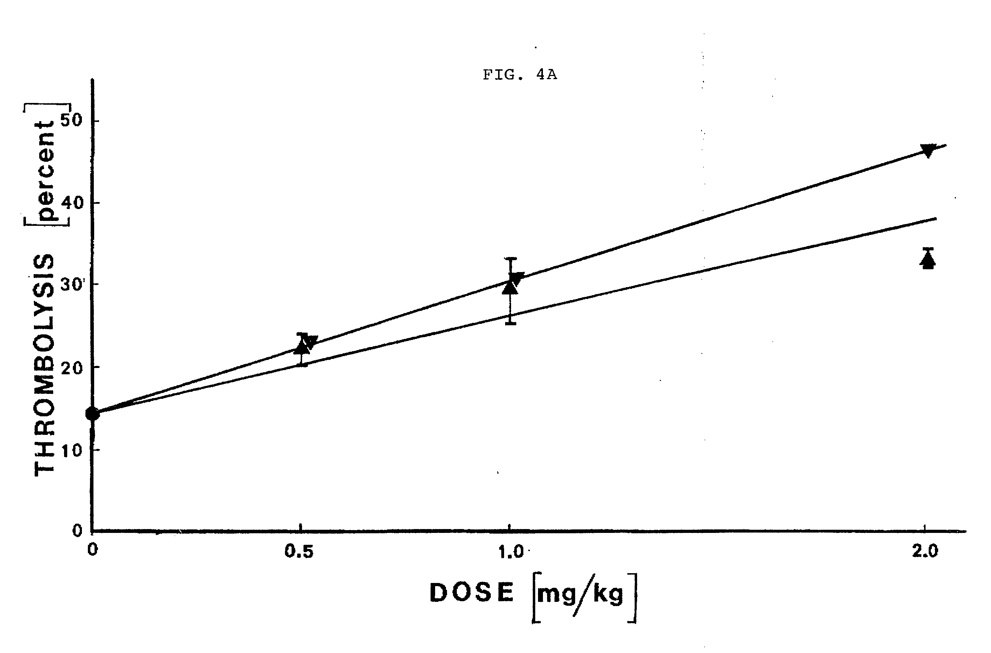

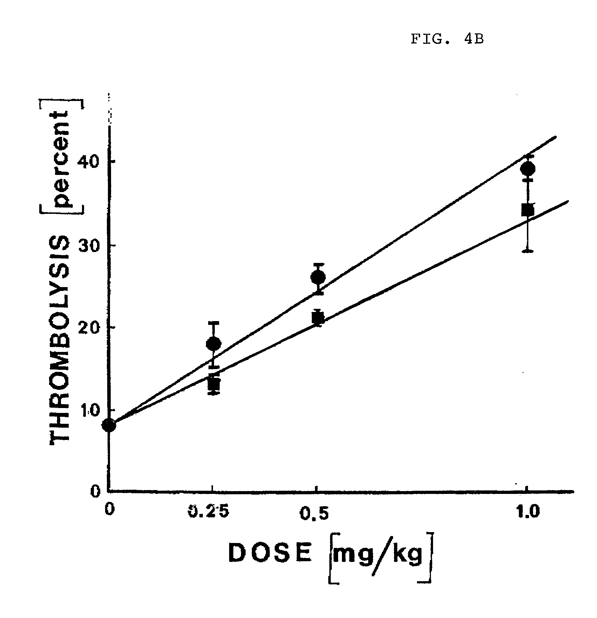

The clearance (in milliliters per min) during the steady-state phase of continuous i.v. infusion was calculated from the ratio between the infusion rate (in μg per min) and the steady-state plasma concentration (in μg/ml). The thrombolytic properties and the fibrin specificity of rscu-PA-His$^{158}$, of nscu-PA and of rscu-PA in a rabbit jugular vein thrombosis model are shown in Table III. Lysis measured 30 minutes after the end of the infusion of solvent into control animals was 14 ± 2 percent. Linear dose-response curves of lysis were observed over a range of 0.5 to 2.0 mg/kg for nscu-PA, rscu-PA and rscu-PA-His$^{158}$ (Fig. 4). At the highest dose used, which yielded 35 to 45 percent lysis, fibrinogen and $\alpha_2$-antiplasmin levels remained unchanged.

Table III

| Thrombolysis after infusion of nscu-PA or rscu-PA-His[158] in rabbits with jugular vein thrombosis. | | | | | |
|---|---|---|---|---|---|
| Agent | Dose (mg/kg) | n | Thrombolysis (percent) | Residual Fibrinogen (percent) | Residual $\alpha_2$-Antiplasmin (percent) |
| Control | - | 3 | 14 ± 2 | 98 ± 1 | 94 ± 4 |
| nscu-PA (Sandoz) | 0.5 | 7 | 22 ± 2 | 91 ± 2 | 86 ± 9 |
| | 1.0 | 7 | 29 ± 4 | 89 ± 2 | 108 ± 4 |
| | 2.0 | 3 | 33 ± 1 | ND | ND |
| rscu-PA | 0.25 | 3 | 18 ± 3 | 95 ± 1 | 89 ± 3 |
| | 0.5 | 3 | 26 ± 2 | 107 ± 6 | 91 ± 8 |
| | 1.0 | 3 | 39 ± 1 | 96 ± 3 | 88 ± 13 |
| nscu-PA (CALU) | 0.25 | 3 | 13 ± 1 | 92 ± 12 | 92 ± 7 |
| | 0.50 | 6 | 21 ± 1 | 97 ± 3 | 85 ± 4 |
| | 1.0 | 4 | 34 ± 5 | 92 ± 2 | 97 ± 4 |
| rscu-PA-His[158] | 0.5 | 2 | 23 | 108 | 94 |
| | 1.0 | 2 | 30 | 91 | 92 |
| | 2.0 | 2 | 46 | 89 | 89 |
| Values represent the mean ± standard error of the mean for nscu-PA and the individual results for rscu-PA-His[158]. | | | | | |

## LEGENDS TO THE FIGURES

Fig. 1: Sodium dodecylsulphate polyacrylamide gel electrophoresis (SDS-PAGE) on 12 percent gels without reduction (A) or after reduction with dithioerythritol (B).
(1) protein calibration mixture consisting of phosphorylase b ($M_r$ 94,000), bovine serum albumin ($M_r$ 67,000, ovalbumin ($M_r$ 43,000), carbonic anhydrase ($M_r$ 30,000), soybean trypsin inhibitor ($M_r$ 30,000) and - lactalbumin ($M_r$ 14,400); (2) rscu-PA-His[158]; (3) rscu-PA-His[158] treated with 7 percent plasmin for 25 min at 37° C; (4): rscu-PA-His[158] treated with 1 percent thrombin for 25 min at 37° C.

Fig. 2: Lysis of a $^{125}$I-fibrin clot immersed in a plasminogen solution, by the single chain forms (A) of u-PA (■), u-PA-Glu[158] (●) and u-PA-His[158] (▲) and their two chain derivatives (B). The data are plotted as percent lysis after 2 hour versus the concentration of plasminogen activator.

Fig. 3: Lysis of $^{125}$I-fibrin labeled hum plasma clots immersed in human plasma by the single chain forms (A) rscu-PA (■), rscu-PA-Glu[158] (●) and rscu-PA-His[158] (▲), and by their two chain derivatives (B). Clot lysis (AI and BI) was measured by the release of soluble 125I-labeled fibrin degradation products and expressed in percent. The data are plotted as percent clot lysis after 2 hours versus the concentration of plasminogen activator.
Residual plasma fibrinogen levels (AII and BII) were measured with a clotting rate assay. Residual fibrinogen after 2 hours is expressed in percent of the baseline value and plotted against the concentration of plasminogen activator. The data represent means of two separate experiments.

Fig. 4: Dose-response of thrombolysis with scu-PA in rabbits with experimental jugular vein thrombosis. The data are expressed as percent clot lysis versus the amount of plasminogen activator (in mg/kg body weight) infused over 4 hours. A. nscu-PA, obtained from Sandoz AG (▲); rscu-PA-His[158] (▼). B. rscu-PA, obtained from Grünenthal (●); nscu-PA, purified from CALU-3 cells (■).

## List of abbreviations

u-PA : urokinase-type plasminogen activator
scu-PA : single chain u-PA
tcu-PA : two chain u-PA, urokinase
nscu-PA : natural scu-PA obtained from a transformed human kidney cell line

rscu-PA : scu-PA obtained by expression of its cDNA in Chinese Hamster ovary cells

rtcu-PA : two chain u-PA obtained by conversion of rscu-PA with plasmin

rscu-PA-His$^{158}$ : rscu-PA, obtained by site-specific mutagenesis of Lys$^{158}$ to His

rscu-PA-Glu$^{158}$: rscu-PA, obtained by substitution of Lys$^{158}$ with Glu

rtcu-PA-Glu$^{158}$ : two chain u-PA-Glu$^{158}$ obtained by digestion of rscu-PA-Glu$^{158}$ with Endoproteinase Glu-C

Glu-plasminogen: native human plasminogen with $NH_2$-terminal glumatic acid

Lys-plasminogen: partially degraded plasminogen with $NH_2$-terminal Lys, Val or Met

S-2444 : pyroglutamyl-glycyl-arginine-p-nitroanilide D-Ile-Pro-Arg-CH$_2$Cl: D-Isoleucyl-prolyl-arginine-chlromethyl-ketone, a synthetic thrombin inhibitor

S-2251 : D-valyl-leucyl-lysine-p-nitroanilide

IU : internation units

KIU : kallikrein inhibitor units

SDS : sodium dodecylsulphate

PAGE : polyacrylamide gel electrophoresis

DTE : dithioerythritol

TNT buffer : Tris-NaCl-Tween buffer ; 0.05 M Tris-HCl buffer pH 7.40 containing 0.038 M NaCl and 0.01% Tween 80

ELISA : enzyme-linked immunosorbent assay

CHO cells : Chinese hamster ovary cells

DHFR : dihydrofolate reductase

## Claims

1. A recombinant human single chain urokinase-type plasminogen activator mutant produced by site-specific mutagenesis of Lys$^{158}$ to His$^{158}$.

2. A recombinant DNA molecule coding for single chain urokinase-type plasminogen activator of which the coding sequence comprises a triplet encoding His$^{158}$.

3. A eukaryotic expression vector containing the recombinant DNA molecule of claim 2.

4. An expression plasmid comprising the eukaryotic expression vector of claim 3.

5. A transfected mammalian cell line comprising the recombinant DNA molecule of claim 2.

6. A method for the preparation of an improved plasminogen activator, wherein said plasminogen activator is recovered of a cell line according to claim 5.

7. A medical composition with thrombolytic activity comprising the recombinant human single chain urokinase-type plasminogen activator mutant according to claim 1 and a pharmaceutically acceptable excipient.

8. In the method for the treatment of patients suffering from thromboembolic disease with an intravenous infusion liquid the improvement which comprises the use of the composition according to claim 7.

1   2   3   4        4   3   2   1

FIG. 1A                FIG. 1B

EP 0 336 508 A1

FIG.2A

FIG.2B

FIG.3A I

FIG.3B I

FIG.3A II

FIG.3B II

Concentration of plasminogen activator (ng/ml)

Concentration of plasminogen activator (µg/ml)

clot lysis after 1 hours

clot lysis after 2 hours

Residual fibrinogen after 2 hours

FIG. 4B

EP 0 336 508 A1

European Patent Office

**PARTIAL EUROPEAN SEARCH REPORT**
which under Rule 45 of the European Patent Convention
shall be considered, for the purposes of subsequent
proceedings, as the European search report

Application number

EP 89200825.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁴) |
|---|---|---|---|
| X | EP - A - 0200541 (GENENTECH. INC.) <br> * abstract; page 7, line 24 - page 9, line 29; page 11, lines 4-29; figure 1; claims 1-5,7,8,11-15,17,20-22,24 * | 1-7 | C12N15/00 <br> C12N9/72 <br> A61K37/00 |
| X | EP - A - 0236040 (COLLABORATIVE RESEARCH INC.) <br> * whole document, in particular page 8, lines 50-57 * | 1-6 | |
| A | CHEMICAL ABSTRACTS <br> vol. 107, no. 17, 26th October 1987, abstract no. 150136 r, Columbus, Ohio, USA; PIERARD LAURENT et al.:"Mutant and chimeric recombinant plasminogen activators. Production in eukaryotic cells and preliminary characterization"; & J. BIOL. CHEM., 1987, vol. 262, no. 24, pages 11771-11778 | 1-6 | |

**TECHNICAL FIELDS SEARCHED (Int. Cl.⁴)**

C12N15/00
C12N9/72
C12P21/02

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7
Claims searched incompletely:
Claims not searched: 8
Reason for the limitation of the search:

Article 52(4) E.P.C.

| Place of search | Date of completion of the search | Examiner |
|---|---|---|
| Berlin | 24.05.89 | P. JULIA Y BALLBE |

EPO Form 1505.1 03 82

European Patent Office

**PARTIAL EUROPEAN SEARCH REPORT**

Application number

EP 89200825.1

| DOCUMENTS CONSIDERED TO BE RELEVANT | | | CLASSIFICATION OF THE APPLICATION (Int. Cl.⁴) |
|---|---|---|---|
| Category | Citation of document with indication, where appropriate, of relevant passages | Relevant to claim | |
| D,A | J. BIOL. CHEM.<br>vol. 262, no. 12, 25th April 1987, Balti-more, US; LUC NELLES et al.:"Characteri-zation of Recombinant Human Single Chain Urokinase-type Plasminogen Activator Mu-tants Produced by Site-specific Mutagene-sis of Lysine 158 "; pages 5682-5689<br>* whole document *<br>--- | 1-6 | |
| | | | TECHNICAL FIELDS SEARCHED (Int. Cl.⁵) |

EPO Form 1505.3  06.78